# EUROPEAN PATENT APPLICATION

(11) **EP 4 019 902 A1**
(43) Date of publication of application: **29.06.2022**
(21) Application number: 21197702.0
(22) Date of filing: 20.09.2021
(51) Int. Cl.: G01C 21/34, G06F 21/32, A61B 5/11

(54) **MULTIMODAL TRIP PLANNING CONSIDERING PHYSIOLOGICAL CAPABILITIES**

(30) Priority: 24.12.2020 US 202017133829
(71) Applicant: Intel Corporation, Santa Clara, CA 95054 (US)
(72) Inventor: POHL, Daniel, 91093 Hessdorf (DE); FOX, Maik, 76287 Rheinstetten (DE)
(74) Representative: Viering, Jentschura & Partner mbB Patent- und Rechtsanwälte

(57) **Abstract**

Various systems and methods for multimodal trip planning are described herein, including determining candidate travel paths for a trip request, determining a score for each of the determined travel candidate paths, and determining adjusted scores for each of the candidate travel paths using an indication of physiological ability of the user.

## Description

### TECHNICAL FIELD

Embodiments described herein generally relate to transportation, and in particular, to multimodal trip planning considering physiological capabilities.

### BACKGROUND

Conventional multimodal trip planning solutions consider geolocation and different modes of transportation in providing users with the fastest or shortest path to a desired location. The fastest or shortest path from an origin or user location to a desired location often involves a pedestrian walking segment, where a user is instructed to proceed on foot between transportation resources or to a transportation resource. Existing solutions predict pedestrian transit time as a measure of determined distance and a predefined average pedestrian walking speed.

### BRIEF DESCRIPTION OF THE DRAWINGS

In the drawings, which are not necessarily drawn to scale, like numerals may describe similar components in different views. Like numerals having different letter suffixes may represent different instances of similar components. Some embodiments are illustrated by way of example, and not limitation, in the figures of the accompanying drawings.
FIG. 1 illustrates an example method of multimodal trip planning.
FIG. 2 illustrates an example method for data acquisition and storage.
FIG. 3 illustrates an example processing platform including a trip planning circuit and associated components and subcomponents.
FIG. 4 illustrates an example multimodal trip planning interface.
FIG. 5 illustrates an example machine in the form of a computer system, within which a set or sequence of instructions may be executed to cause the machine to perform any one of the methodologies discussed herein.

### DETAILED DESCRIPTION

In the following description, for purposes of explanation, numerous specific details are set forth in order to provide a thorough understanding of some example embodiments. It will be evident, however, to one skilled in the art that the present disclosure may be practiced without these specific details.

Multimodal trip planning requires consideration of many possible modes of transportation to optimize travel for a user between an origin and destination to meet certain goals. For example, time of or between transportation modes can be extended to account for slower user walking speed. The present inventors have recognized, among other things, that different transportation modes have different physical requirements from the user, and that, given differences between users, the best option for a particular user may be particular to that individual user. Accordingly, transportation modes and navigation instructions can be optimized or adjusted to reduce physiological stress of travel for particular users based on certain information from the particular users.

Different modes of public transportation can be physiologically stressful to different users. For certain users, a short walk over a flat distance between transportation modes is vastly different than a short distance between transportation modes with an unmapped vertical relief, such as a flight of stairs, hill, or incline. Multimodal trips are often more susceptible to stress than single-mode transportation, as navigation or routing instructions of multimodal trips are often connected in series, and missing one connection time can have cascading effects on the overall trip, increasing the urgency and stress of each segment.

For example, a short time period between connecting train segments may require the user to move quickly to make the planned or routed connection. In other examples, navigation instructions may route users up staircases without warning or adequate scheduling to re-route without impacting shared connections. The user may find themselves directed to bicycle or e-scooter segments at times of busy traffic.

The present inventors have recognized, among other things, that stresses can be managed, reduced, or avoided during multimodal trip planning using crowdsourced and individual data, such as, for example, indications of user physiological condition or capabilities, user preferences, or received user information.

In certain examples, stresses can be managed such as by allowing additional time between segments or adjusting (e.g., reducing) average speeds of specific transit based on (e.g., commensurate with) topography, elevation change, environmental factors, air quality indicators, daily physiological information, or trends of daily physiological information, such as short-term (e.g., 3-day averages, 5-day averages, etc.) or long-term trends (e.g., 2-week averages, monthly-averages, etc.), etc. Stresses can be reduced or avoided such as by re-routing the user around physical impediments or adjusting routing to avoid known stressful conditions or situations, including avoiding stairs, selecting segments with a moderate average grade, avoiding specific modes of travel (e.g., walk, bike, e-bike, e-scooter, vehicle (including a ride-sharing vehicle, taxi, or partial or fully-autonomous vehicle, etc.), bus, train, etc.) in specific instances, such as using information stored in a user profile, crowdsourced information (e.g., relative to user information), etc. In other examples, relative stresses can be determined as estimated difficulties and displayed to the user on a scale, such as either easy or difficult, or given a relative score (e.g., between 0 (easy) and 100 (difficult), etc.) with respect to other candidate routes or routes or segments from a history of the user.

In additional examples, environmental data can be used for further optimization of speeds, routes, or mode of travel. Taking physiological capabilities into consideration during multimodal trip planning, and additionally environmental factors, can improve ridership and experience of multimodal trip planning over a wider range of multimodal transportation options, optimizing planning, selection, prioritization, and usage of transportation resources.

Traditional navigation and routing use map data from a central database, as well as traffic information, etc., to determine candidate travel paths for a user. The multimodal trip planning circuit disclosed herein can augment traditional navigation and routing by considering additional information, such as information about the physiological ability of the user, user preferences, environmental factors, travel experience, etc., to provide a personalized public transport experience.

Environmental factors can include one or more weather factors, such as temperature (e.g., heat, cold, windchill, heat index, etc.), sun, rain, wind, humidity, etc. In other examples, the environmental factors can include one or more air quality indicators, such as indications of pollutants, smog, pollen, dust, sand, etc. In certain examples, the environmental factors can include one or more weather factors in combination with one or more air quality indicators, such as sensed by one or more sensors of the mobile device or stored in a database available to the multimodal trip planning circuit, such as corresponding to a location of the user, the starting location, the destination, or the general area and time of the determined route or trip request, etc.

Travel experience can include a user experience or history with the particular route, similar routes, or user experience with the determined candidate modes of transport. In other examples, crowdsourced information, such as stored in a database available to the multimodal trip planning circuit, can be used, such as in the absence of user experience or history for a particular route or mode of transport. In an example, crowdsourced information corresponding to segments without user history or experience can be normalized with other segments having information of both the user and crowdsourced information.

Multimodal trip planning has multiple degrees of freedom and optimization goals. To improve the user experience, route planning for the user can be determined using information about the physiological ability and preferences of the user, in certain examples, relative to environmental factors, such as weather (e.g., heat, sun, rain, cold, humidity, etc.), air quality indicators (e.g., pollutants, smog, pollen, dust, sand, etc.), etc.

In certain examples, the user can preselect preferences, which can be stored in a user profile. In other examples, the user profile can be created using past trip information, either in combination with physiological information, such as from a smart watch, chest strap, or other wearable sensor configured to be worn or used by the user incident to or close in time with multimodal trips or segments of multimodal trips, or user ratings or indications of user experience associated with multimodal trips or segments of multimodal trips, or the past trip information itself, such as selecting alternative routes, trends of selected modes incident to environmental factors, days of the week, time of day, season, etc.

In an example, to protect user information, such as for data privacy, only general information can be recorded, such as a relative slope of heart rate changes (e.g., recovery), change in user speed with elevation changes, grade, etc. In certain examples, to maintain data privacy, general information can be stored in databases outside the mobile device or outside the user profile, and such general information can be normalized on the mobile device using user specific information, such that specific patient physiological information is not uploaded into the cloud.

In an example, a mobility application on a mobile device of the user, such as a smartphone, can have access to activity and health data of the user associated with the mobile device or one or more components coupled thereto, such as a smartwatch, fitness tracker, motion sensors, elevation sensors, etc., after receiving appropriate consent. Physiological information can be collected anonymously by the mobility application, either locally or in the cloud, and averaged for route segments based on a mode of transportation, including walking between vehicle segments, etc.

A multimodal trip planning circuit can receive a trip request to route a user to a destination, such as through a user input to user interface of a mobility application on a mobile device. During an initial multimodal trip planning phase, the user can select a certain expectation on the "comfort level" of the trip ahead (e.g., it's hot and the user wants to avoid sweating too much, so a "high comfort level" can be selected). This information is used as an additional optimization goal for the trip planning. Modes of transportation for various segments can be scored, and such scoring can be weighted if the mode of transportation requires user motion or activity (e.g. bike, e-bike, e-scooter, or walking) and one or more stresses are preset. Weightings can further be adjusted based on the activity and health data of past users travelling that segment. In certain examples, steep up-hill bike rides (e.g., greater than 4%), steep uphill walking segments (e.g., greater than a 6% grade, etc.), multi-floor staircases, etc., can be avoided if the user does not feel like it today or is not physiologically capable of it. In other examples, users desiring activity can request or prioritize it, such as in the trip request, leading to a kind of merging concept of combining transportation with a workout.

In an example, candidate travel paths can be broken into candidate segments and scored. Scoring of candidate segments, such as described herein, can take multiple factors into account, such as: a mode of transport for a trip segment; an activity factor of a trip segment; or personal preferences.

The mode of transport can provide different impact to various other factors. For example, exposure to the environment increases the impact of environmental factors, such as adverse weather (e.g., heat, rain) or pollen, etc. Further, the physical stress on the user can be different, for example, biking versus vehicle.

The activity factor of a trip segment is often incomplete in standard mapping data. Activities or segments such as walking to a vehicle (e.g., a ride-sharing vehicle, taxi, or partial or fully-autonomous vehicle, etc.) or changing between trains or from a train to a e-scooter have a very individual activity factors. Usually only the distance for a walking segment is given. The more important factors are elevation and, in the case of staircases, the number of steps. If detailed maps and data are available (e.g., in a train station) for walking routes, such as from platform A to B (e.g., number of stairs, distance, elevation, etc.), an activity factor for that segment can be calculated as a factor and augmented later by individual data on walking segments. If there is no detailed data, crowdsourced or individual past trip data from customers can be used to calculate the score and make an estimated activity factor determination.

Personal preferences can be determined and scored separately. The user can provide indications of severity of impact of various conditions, such as pollen allergies, sun exposure, limited physical abilities (e.g., issues walking longer stretches, injuries, permanent disability, etc.), unwillingness to ride e-scooters, no bike riding during the rain, etc.

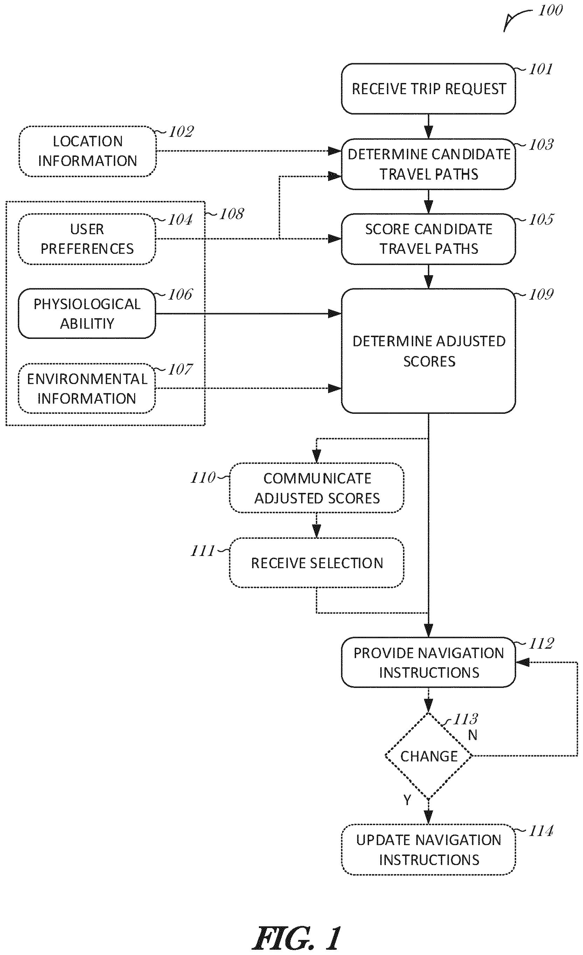

FIG. 1 illustrates an example method 100 of providing multimodal trip planning considering physiological capabilities, such as through a mobility application of a mobile device. A web interface is possible, such as through a laptop or desktop computer, but to collect data during the trip and offer dynamic trip adjustments, such as by updating navigation instructions, sensors of a mobile device, such as a smartphone or a physiological sensor of a wearable device (e.g., a smartwatch, fitness monitor, etc.).

At operation 101, a trip request can be received, such as a trip request to route a user to a destination at a trip planning circuit. In an example, the trip request can be a multimodal trip request. In certain examples, the trip request can include information about not only the destination, but the type of trip (e.g., business, personal, leisure, exercise, carrying packages or groceries, etc.). At operation 102, location information can be received, such as using one or more location circuits of a mobile or wearable device. At operation 103, candidate travel paths for the trip request can be determined using location information of the user, such as using a navigation circuit. In certain examples, other information in the trip request can be used to determine or select candidate travel paths, such as the type of trip, etc.

At operation 104, user preferences can be received, such as preferred modes of travel, experience with modes of travel, schedules, preferred modes of travel with respect to environmental factors, etc. In an example, the candidate travel paths can be determined at operation 103 using the received user preferences.

At operation 105, scores for the candidate travel paths can be determined, such as using the navigation circuit. In an example, the scores can be indicative of an estimated travel time of respective candidate travel paths for an average user, such as with respect to an average transit speed for each mode of travel. In an example, the candidate travel paths can include a number of the top candidates (e.g., 3, 4, 10, etc.). In the simplest form, scores are indicative of the estimated travel time, and are a function of the distance between a starting point or a current location of the user and a destination, divided by an average transit speed for the specific mode of travel.

At operation 106, indications of physiological ability of the user can be received, such as indications of physiological information (e.g., heart rate, blood pressure, activity information, etc.) or other indications as described herein. For example, anonymized and individual physical activity profiles can be received and used to score candidate travel paths. At operation 107, environmental information of the user can be received, such as weather or one or more air quality indicators, such as indications of pollutants, smog, pollen, dust, sand, etc. At operation 108, one or more of the user preferences, physiologic ability, and environmental information can be used to determine a user profile. In an example, the user profile can include multimodal planning goals with preferred trip segments. For example, following trips or segments, the user can be prompted to provide feedback on the trip or portions of the trip. The feedback can be used to adjust user preferences going forward, such as to encourage segments having a higher rating or that most closely align with information from the trip request.

At operation 109, adjusted scores can be determined for each of the candidate travel paths. The adjusted scores can be determined for each of the candidate travel paths using received indications of physiological ability of the user. In one example, the received indications of physiological ability can be used to weight one or more aspects of the function used to determine the score at operation 105. For example, the adjusted scores can be indicative of an as estimated difficulty of the respective candidate travel path, such as with respect to the average user, or a relative score (e.g., between 0 (easy) and 100 (difficult), etc.) with respect to other candidate routes or routes or segments from a history of the user. The adjusted scores can be determined using the indication of physiological ability, such as to increase or decrease the score as a function of the physiological ability of the user with respect to an average user. Indications of physiological ability can include, among other things, average user walking speed, average speed of elevation changes, heart rate information, heart rate recovery, or one or more other measures, such as in contrast to average measures of a group of users

At operation 110, one or more of the adjusted scores can be communicated to the user, such as presenting an indication of one or more of the adjusted scores to the user on a user interface of a mobile device. In an example, an indication representative of a time of the adjusted scores can be displayed, or an ease or difficulty of travel associated with a particular candidate travel path. At operation 111, a selection can be received, such as from the user in response to communication of the adjusted scores, through the user interface of the mobile device.

At operation 112, navigation instructions can be provided to the user. In certain examples, given one or more aspect of the user profile 108, navigation instructions having and adjusted score better than the other scores by a factor, or consistent with previously highly rated parameters by the user can be provided without separate communication and selection.

At operation 113, if one or more aspects of the user profile changes beyond a threshold (e.g., a percent change, such as greater than 20%, etc.), such as weather, pollen forecasts, or physiological information (e.g., user heart rate or activity data increases beyond that expected, etc.), the navigation instructions can be updated at operation 114, such as by returning to operation 103 and determining new scores for candidate travel paths given the changed parameters. For example, if the heart rate of a user on a biking segment is higher than anticipated (e.g., greater than an activity threshold for moderate exercise, such as 50% of max heart rate, where max heart rate is 220-age), a new multimodal trip can be calculated including adjustments in the background. If a new score is better than the current trip score, given the changes, then a new path can be determined and communicated to the user. The user can then be prompted to decide to continue or transition modes. In other examples, if it starts raining while on a walking or biking segment, the user can be prompted to transition to a bus, train, or vehicle (e.g., a ride-sharing vehicle, taxi, or partial or fully-autonomous vehicle, etc.) segment.

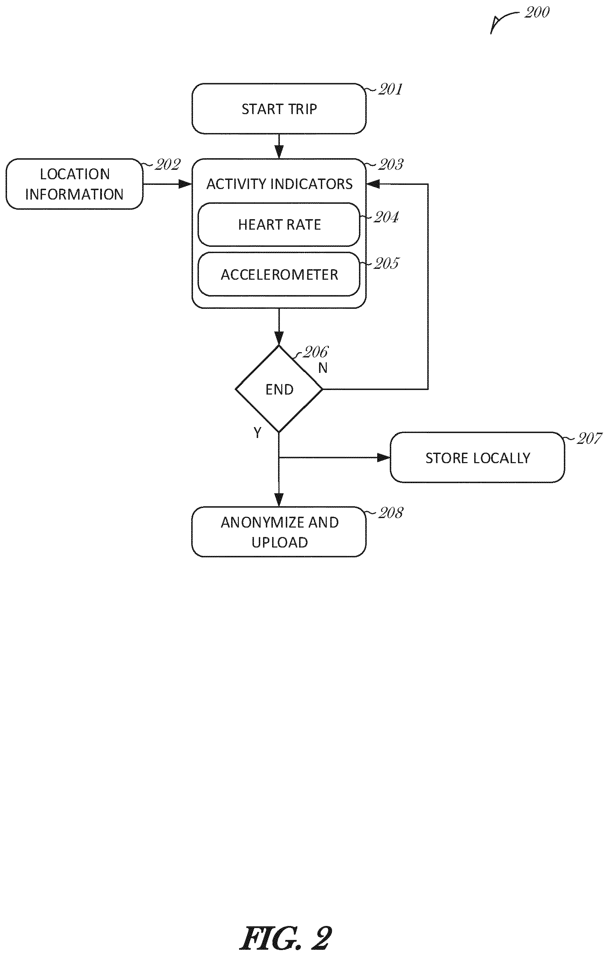

FIG. 2 illustrates an example method for data acquisition and storage while on a trip. Score planning or determination, as disclosed herein, can occur in the cloud or locally on the mobile device. There are benefits to performing calculations on the mobile device, especially in the context of considering individual health data of the user.

At operation 201, a trip can start, such as by receiving a trip request. At operation 202, location information can be received, such as GPS or other cellular or wireless data connection location information at a mobile device or wearable device. At operation 203, one or more activity indicators can be received or determined, such as from one or more sensors of the mobile device or wearable physiological sensor (e.g., from a smartwatch or fitness monitor, etc.). Heart rate information can be received or determined at operation 204. Accelerometer or activity information can be received or determined at operation 205. At operation 206, if the end of a trip is determined, profile information can be updated using the activity information from the trip at operation 207. At operation 208, data can be anonymized, such as by removing specific data and separating such from the user, and uploading such data to the cloud or one or more other databases to improve candidate travel path determination or overall models for multimodal trip planning, without compromising data privacy.

In an example, traditional segment scoring can be performed in the cloud away from the device, while adjusted scores that take into account specific user data can be performed locally to maintain data privacy. The more feedback the user provides, the better the scoring becomes for the particular user. Accordingly, networked processes and databases can perform traditional scoring route planning, adapted by specific user preferences and the user profile created on the device.

The user profile can further include a physiological ability factor determined over time for the particular user. A user can start as an average user, in certain examples adapted by one or more of age, sex, height, or weight. After each trip, the average activity levels can be compared to the data available in a health database, such as using a trip planning circuit or one or more other processors or circuits. The ability factor can be adjusted slightly depending on if the customer is above or below average activity levels compared to the crowd. The ability factor can be scored on a scale, or can include one or more categories, such as average (e.g., within a standard deviation from the mean, etc.), below average, or above average, etc. The ability factor can be used for future trip planning scoring.

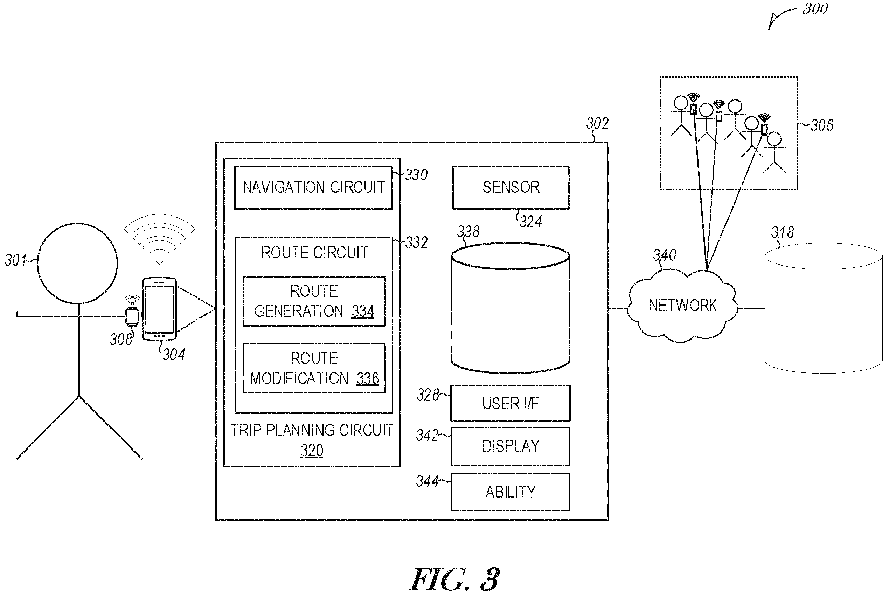

FIG. 3 illustrates an example processing platform 302, such as of a mobile device 304 and a wearable physiological sensor 308 of a user 301, the processing platform 302 including a trip planning circuit 320 and associated components and subcomponents, including a user interface 328 configured to receive information from the user 301 (or other users, such as friends, family members, or connected users, such as through a mobile application, etc.), a navigation circuit 330 configured to, among other things, determine at least one candidate travel path for a user, provide navigation guidance to a user, a route circuit 332 comprising a route generation circuit 334 and a route modification circuit 336 configured to determine candidate routes and alter such based on input from or preferences of a user or groups of users, or physiological information about the user or environmental information of the route, and a display 342 configured to provide information to the user 301.

In an example, the processing platform 302 can determine a location, speed, and average speed of the user 301 or otherwise receive inertial, accelerometer, or location information, such as from a sensor 324, etc. Information about navigation, routes, the user 301, etc., can be stored in a database 338. Information, such as routing information or options can be provided to the user 301 using a display 342. The processing platform 302 can further be coupled to a remote database 318 external to the processing platform 302 or the mobile device 304, such as through a network 340, configured to store or contain different information from one or more other users, geographic mapping information, etc. In an example, the navigation circuit 330 can be a component of the network 340, such as implemented on a remote circuit or processing platform available via the network 340 using geographic or mapping information stored in the remote database 318.

In an example, one or more other users 306 can be coupled to the network 340. Information or communication from the one or more other users 306 can be used to determine a multimodal trip plan. The wearable physiological sensor 308 can include a smartwatch, a fitness monitor, a GPS watch, or one or more other wearable devices configured to monitor the physical activity of a user. The processing platform 302 can further include a physiological ability circuit 344 configured to determine an indication of patient physiological ability, such as, using physiological information from or about the user. In certain examples, the determined indication of physical ability can include that the user

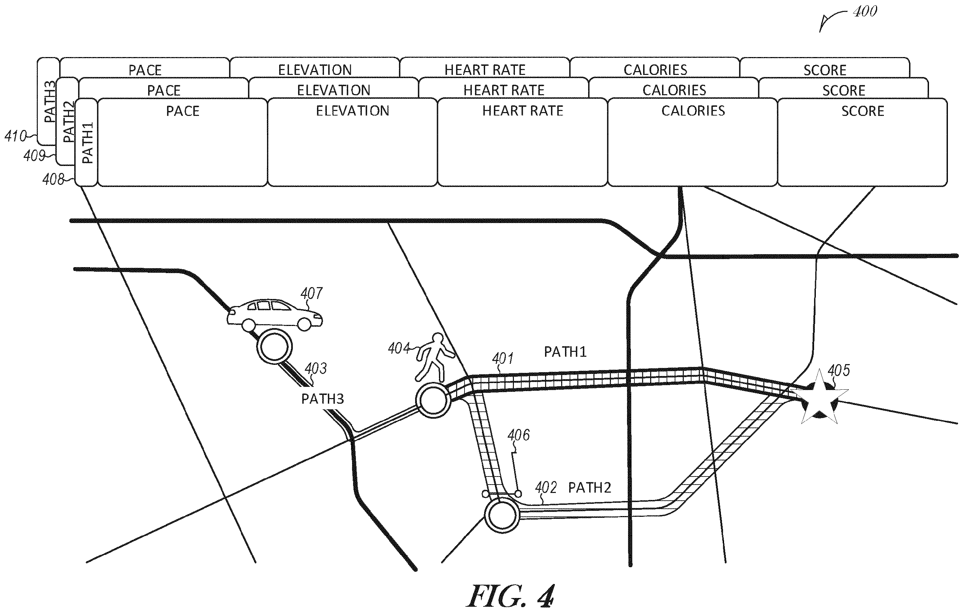

FIG. 4 illustrates an example multimodal trip planning interface 400, including candidate first, second, and third travel paths 401, 402, 403, and respective first, second, and third displays 408, 409, 410, including information for each path, in certain examples specific to a user 404, including scores for each travel path. Although illustrated herein as a short distance between a location of the user 404 and a destination 405, many paths will be much longer, including travel options having or requiring multiple modes of travel, such as walk, bike, e-scooter, vehicle (e.g., a ride-sharing vehicle, taxi, or partial or fully-autonomous vehicle, etc.), bus, or train, etc.

The first travel path 401 is a walking path from the location of the user 404 to the destination 405. An average score for the first travel path 401 can be based on an average walking time of the first travel path 401 for a plurality of users in contrast to a product of an average walking speed of the plurality of users and the distance. In an example, the first travel path 401 can include a large elevation change. If the indication of physiological ability of the user 404, such as an ability factor received or determined with respect to a plurality of users, indicates that the ability of the user 404 is below average, or if the user 404 has provided information, such as user preferences, to avoid large elevation gains or combinations of elevation gains and environmental factors (e.g., weather, pollution, etc.), etc., an adjusted score can be determined, such as weighting the average score by the indication of physiological ability, decreasing the average score.

The second candidate travel path 402 comprises two segments: a walk segment from the location of the user 404 to a location of an e-scooter 406; and an e-scooter segment from the e-scooter 406 to the destination 405. The relative difficulty of an e-scooter mode of travel can be less than a walking mode, especially with segments having positive elevation gain. The score of the second travel path 402 can be higher than the score of the first travel path 401.

The third travel path 403 comprises a vehicle segment from a location of a vehicle 407 to the location of the user 404, then proceeding to the destination 405 in the vehicle 407. In an example, the vehicle can include a taxi, a ride-sharing vehicle, or a partial or fully-autonomous vehicle. The relative difficulty of a vehicle mode of travel can be lower than a walking mode or an e-scooter mode. However, if a user profile includes preferences, such as in certain environmental conditions (e.g., weather, pollution, etc.), the score of the third travel path 403 can be lower than the second travel path 402, but higher than the first travel path 401.

The user can select a desired path in response to the provided the scores, and instructions can be provided for the selected path. In certain examples, once selected, reservations for the selected mode of travel can be made.

Embodiments may be implemented in one or a combination of hardware, firmware, and software. Embodiments may also be implemented as instructions stored on a machine-readable storage device, which may be read and executed by at least one processor to perform the operations described herein. A machine-readable storage device may include any non-transitory mechanism for storing information in a form readable by a machine (e.g., a computer). For example, a machine-readable storage device may include read-only memory (ROM), random-access memory (RAM), magnetic disk storage media, optical storage media, flash-memory devices, and other storage devices and media.

A processor subsystem may be used to execute the instruction on the -readable medium. The processor subsystem may include one or more processors, each with one or more cores. Additionally, the processor subsystem may be disposed on one or more physical devices. The processor subsystem may include one or more specialized processors, such as a graphics processing unit (GPU), a digital signal processor (DSP), a field programmable gate array (FPGA), or a fixed function processor.

Examples, as described herein, may include, or may operate on, logic or a number of components, modules, or mechanisms. Modules may be hardware, software, or firmware communicatively coupled to one or more processors in order to carry out the operations described herein. Modules may be hardware modules, and as such modules may be considered tangible entities capable of performing specified operations and may be configured or arranged in a certain manner. In an example, circuits may be arranged (e.g., internally or with respect to external entities such as other circuits) in a specified manner as a module. In an example, the whole or part of one or more computer systems (e.g., a standalone, client or server computer system) or one or more hardware processors may be configured by firmware or software (e.g., instructions, an application portion, or an application) as a module that operates to perform specified operations. In an example, the software may reside on a machine-readable medium. In an example, the software, when executed by the underlying hardware of the module, causes the hardware to perform the specified operations. Accordingly, the term hardware module is understood to encompass a tangible entity, be that an entity that is physically constructed, specifically configured (e.g., hardwired), or temporarily (e.g., transitorily) configured (e.g., programmed) to operate in a specified manner or to perform part or all of any operation described herein. Considering examples in which modules are temporarily configured, each of the modules need not be instantiated at any one moment in time. For example, where the modules comprise a general-purpose hardware processor configured using software; the general-purpose hardware processor may be configured as respective different modules at different times. Software may accordingly configure a hardware processor, for example, to constitute a particular module at one instance of time and to constitute a different module at a different instance of time. Modules may also be software or firmware modules, which operate to perform the methodologies described herein.

As used in any embodiment herein, the term "logic" may refer to firmware and/or circuitry configured to perform any of the aforementioned operations. Firmware may be embodied as code, instructions or instruction sets and/or data that are hard-coded (e.g., nonvolatile) in memory devices and/or circuitry.

"Circuitry," as used in any embodiment herein, may comprise, for example, singly or in any combination, hardwired circuitry, programmable circuitry, state machine circuitry, logic and/or firmware that stores instructions executed by programmable circuitry. The circuitry may be embodied as an integrated circuit, such as an integrated circuit chip. In some embodiments, the circuitry may be formed, at least in part, by the processor circuitry executing code and/or instructions sets (e.g., software, firmware, etc.) corresponding to the functionality described herein, thus transforming a general-purpose processor into a specific-purpose processing environment to perform one or more of the operations described herein. In some embodiments, the processor circuitry may be embodied as a stand-alone integrated circuit or may be incorporated as one of several components on an integrated circuit. In some embodiments, the various components and circuitry of the node or other systems may be combined in a system-on-a-chip (SoC) architecture

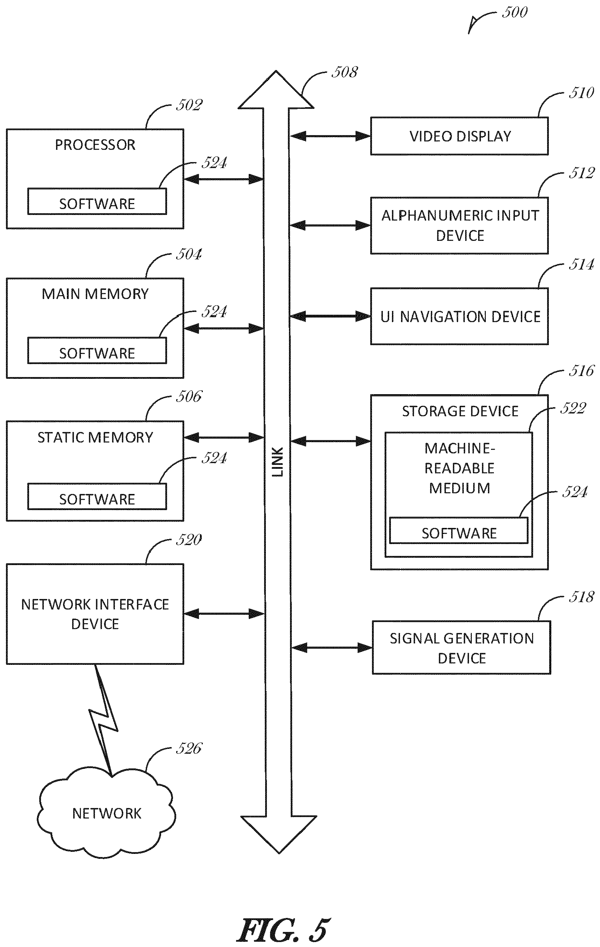

FIG. 5 illustrates an example machine in the form of a computer system 500, within which a set or sequence of instructions may be executed to cause the machine to perform any one of the methodologies discussed herein. In alternative embodiments, the machine operates as a standalone device or may be connected (e.g., networked) to other machines. In a networked deployment, the machine may operate in the capacity of either a server or a client machine in server-client network environments, or it may act as a peer machine in peer-to-peer (or distributed) network environments. The machine may be a vehicle subsystem, a personal computer (PC), a tablet PC, a hybrid tablet, a personal digital assistant (PDA), a mobile telephone, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein. Similarly, the term "processor-based system" shall be taken to include any set of one or more machines that are controlled by or operated by a processor (e.g., a computer) to individually or jointly execute instructions to perform any one or more of the methodologies discussed herein.

Example computer system 500 includes at least one processor 502 (e.g., a central processing unit (CPU), a graphics processing unit (GPU) or both, processor cores, compute nodes, etc.), a main memory 504 and a static memory 506, which communicate with each other via a link 508 (e.g., bus). The computer system 500 may further include a video display unit 510, an alphanumeric input device 512 (e.g., a keyboard), and a user interface (UI) navigation device 514 (e.g., a mouse). In one embodiment, the video display unit 510, input device 512 and UI navigation device 514 are incorporated into a touch screen display. The computer system 500 may additionally include a storage device 516 (e.g., a drive unit), a signal generation device 518 (e.g., a speaker), a network interface device 520, and one or more sensors (not shown), such as a global positioning system (GPS) sensor, compass, accelerometer, gyrometer, magnetometer, or other sensor.

The storage device 516 includes a machine-readable medium 522 on which is stored one or more sets of data structures and instructions 524 (e.g., software) embodying or utilized by any one or more of the methodologies or functions described herein. The instructions 524 may also reside, completely or at least partially, within the main memory 504, static memory 506, and/or within the processor 502 during execution thereof by the computer system 500, with the main memory 504, static memory 506, and the processor 502 also constituting machine-readable media.

While the machine-readable medium 522 is illustrated in an example embodiment to be a single medium, the term "machine-readable medium" may include a single medium or multiple media (e.g., a centralized or distributed database, and/or associated caches and servers) that store the one or more instructions 524. The term "machine-readable medium" shall also be taken to include any tangible medium that is capable of storing, encoding or carrying instructions for execution by the machine and that cause the machine to perform any one or more of the methodologies of the present disclosure or that is capable of storing, encoding or carrying data structures utilized by or associated with such instructions. The term "machine-readable medium" shall accordingly be taken to include, but not be limited to, solid-state memories, and optical and magnetic media. Specific examples of machine-readable media include non-volatile memory, including but not limited to, by way of example, semiconductor memory devices (e.g., electrically programmable read-only memory (EPROM), electrically erasable programmable read-only memory (EEPROM)) and flash memory devices; magnetic disks such as internal hard disks and removable disks; magneto-optical disks; and CD-ROM and DVD-ROM disks.

The instructions 524 may further be transmitted or received over a communications network 526 using a transmission medium via the network interface device 520 utilizing any one of a number of well-known transfer protocols (e.g., HTTP). Examples of communication networks include a local area network (LAN), a wide area network (WAN), the Internet, mobile telephone networks, plain old telephone (POTS) networks, and wireless data networks (e.g., Bluetooth, Wi-Fi, 3G, and 4G LTE/LTE-A, 5G, DSRC, LoRa/LoRaWAN, or satellite communication networks). The term "transmission medium" shall be taken to include any intangible medium that is capable of storing, encoding, or carrying instructions for execution by the machine, and includes digital or analog communications signals or other intangible medium to facilitate communication of such software.

The above detailed description includes references to the accompanying drawings, which form a part of the detailed description. The drawings show, by way of illustration, specific embodiments that may be practiced. These embodiments are also referred to herein as "examples." Such examples may include elements in addition to those shown or described. However, also contemplated are examples that include the elements shown or described. Moreover, also contemplated are examples using any combination or permutation of those elements shown or described (or one or more aspects thereof), either with respect to a particular example (or one or more aspects thereof), or with respect to other examples (or one or more aspects thereof) shown or described herein.

Example 1 is a system comprising: a trip planning circuit configured to receive a trip request to route a user to a destination, and to receive location information of the user; and a navigation circuit configured to determine candidate travel paths for the trip request using the destination and location information of the user, and to determine a score for each of the determined travel candidate paths; wherein the trip planning circuit is configured to receive an indication of physiological ability of the user, and wherein the trip planning circuit is configured to determine adjusted scores for each of the candidate travel paths using the received indication of physiological ability of the user and to communicate at least one of the adjusted scores to the user.

In Example 2, the subject matter of Example 1 includes, wherein to determine the score for each of the determined travel candidate paths, the navigation circuit is configured to determine the score for each of the determined travel candidate paths indicative of an estimated travel time for each respective candidate travel path for an average user.

In Example 3, the subject matter of Example 2 includes, wherein the adjusted scores for each of the candidate travel paths are indicative of at least one of an adjusted estimated travel time or an estimated difficulty for each respective candidate travel path for the user with respect to the received indication of physiological ability of the user.

In Example 4, the subject matter of Examples 1-3 includes, a physiological ability circuit configured to determine the indication of physiological ability of the user using at least one of heart rate or activity information of the user.

In Example 5, the subject matter of Examples 1-4 includes, wherein the trip planning circuit is configured to receive environmental information associated with the trip request, and wherein the trip planning circuit is configured to determine the adjusted scores for each of the candidate travel paths using the received indication of physiological ability of the user and the environmental information.

In Example 6, the subject matter of Example 5 includes, wherein the environmental information comprises at least one of pollution information or weather information associated with a start time and a location of the trip request.

In Example 7, the subject matter of Examples 1-6 includes, a mobile device including the trip planning circuit, the mobile device comprising a smartphone; and a wearable physiological sensor configured to receive one of heart rate information and activity information of the user.

In Example 8, the subject matter of Example 7 includes, wherein the trip planning circuit is configured to receive the indication of physiological ability of the user from the wearable physiological sensor or the mobile device.

In Example 9, the subject matter of Examples 7-8 includes, wherein the trip planning circuit is configured, after the user arrives at the destination, to update a user profile with physiological information of the user associated with the trip request, to anonymize the physiological information of the user associated with the trip request, and to upload the anonymized physiological information to a remote database.

In Example 10, the subject matter of Example 9 includes, wherein the trip planning circuit is configured to receive the indication of physiological ability of the user from the user profile.

Example 11 is a method comprising: receiving a trip request to route a user to a destination; receiving location information of the user; determining candidate travel paths for the trip request using the destination and location information of the user; determining a score for each of the determined travel candidate paths; receiving an indication of physiological ability of the user, determining adjusted scores for each of the candidate travel paths using the received indication of physiological ability of the user; and communicating at least one of the adjusted scores to the user.

In Example 12, the subject matter of Example 11 includes, wherein determining the score for each of the determined travel candidate paths comprises determining an indication of an estimated travel time for each respective candidate travel path for an average user.

In Example 13, the subject matter of Example 12 includes, wherein determining adjusted scores for each of the candidate travel paths comprises determining an indication of at least one of an adjusted estimated travel time or an estimated difficulty for each respective candidate travel path for the user with respect to the received indication of physiological ability of the user.

In Example 14, the subject matter of Examples 11-13 includes, determining the indication of physiological ability of the user using at least one of heart rate or activity information of the user.

In Example 15, the subject matter of Examples 11-14 includes, receiving environmental information associated with the trip request comprising at least one of pollution information or weather information associated with a start time and a location of the trip request; and determining the adjusted scores for each of the candidate travel paths using the received indication of physiological ability of the user and the environmental information.

In Example 16, the subject matter of Examples 11-15 includes, wherein receiving the trip request, determining adjusted scores, and communicating at least one of the adjusted scores to the user comprises using a smartphone, and wherein receiving the indication of physiological ability of the user comprises receiving one of heart rate information or activity information of the user using a wearable physiologic sensor.

In Example 17, the subject matter of Example 16 includes, updating a user profile with physiological information of the user associated with the trip request after the user arrives at the destination; and anonymizing the physiological information of the user associated with the trip request; and uploading the anonymized physiological information to a remote database.

Example 18 is at least one non-transitory machine-readable medium including instructions for multimodal trip planning, the instructions, when executed by hardware circuitry, cause the hardware circuitry to perform operations comprising: receiving a trip request to route a user to a destination; receiving location information of the user; determining candidate travel paths for the trip request using the destination and location information of the user; determining a score for each of the determined travel candidate paths; receiving an indication of physiological ability of the user, determining adjusted scores for each of the candidate travel paths using the received indication of physiological ability of the user; and communicating at least one of the adjusted scores to the user.

In Example 19, the subject matter of Example 18 includes, wherein determining the score for each of the determined travel candidate paths comprises determining an indication of an estimated travel time for each respective candidate travel path for an average user.

In Example 20, the subject matter of Example 19 includes, wherein determining adjusted scores for each of the candidate travel paths comprises determining an indication of at least one of an adjusted estimated travel time or an estimated difficulty for each respective candidate travel path for the user with respect to the received indication of physiological ability of the user.

In Example 21, the subject matter of Examples 18-20 includes, the operations comprising: determining the indication of physiological ability of the user using at least one of heart rate or activity information of the user.

In Example 22, the subject matter of Examples 18-21 includes, the operations comprising: receiving environmental information associated with the trip request comprising at least one of pollution information or weather information associated with a start time and a location of the trip request; and determining the adjusted scores for each of the candidate travel paths using the received indication of physiological ability of the user and the environmental

Example 1 is a system comprising: a trip planning circuit configured to receive a trip request to route a user to a destination, and to receive location information of the user; and a navigation circuit configured to determine candidate travel paths for the trip request using the destination and location information of the user, and to determine a score for each of the determined travel candidate paths; wherein the trip planning circuit is configured to receive an indication of physiological ability of the user, and wherein the trip planning circuit is configured to determine adjusted scores for each of the candidate travel paths using the received indication of physiological ability of the user and to communicate at least one of the adjusted scores to the user.

In Example 2, the subject matter of Example 1 includes, wherein to determine the score for each of the determined travel candidate paths, the navigation circuit is configured to determine the score for each of the determined travel candidate paths indicative of an estimated travel time for each respective candidate travel path for an average user.

In Example 3, the subject matter of Example 2 includes, wherein the adjusted scores for each of the candidate travel paths are indicative of at least one of an adjusted estimated travel time or an estimated difficulty for each respective candidate travel path for the user with respect to the received indication of physiological ability of the user.

In Example 4, the subject matter of Examples 1-3 includes, a physiological ability circuit configured to determine the indication of physiological ability of the user using at least one of heart rate or activity information of the user, wherein the trip planning circuit is configured to determine adjusted scores for each of the candidate travel paths as a function of the indication of physiological ability of the user compared to an average physiological ability of a group of users.

In Example 5, the subject matter of Examples 1-4 includes, wherein the trip planning circuit is configured to receive environmental information associated with the trip request, and wherein the trip planning circuit is configured to determine the adjusted scores for each of the candidate travel paths using the received indication of physiological ability of the user and the environmental information.

In Example 6, the subject matter of Example 5 includes, wherein the environmental information comprises at least one of pollution information or weather information associated with a start time and a location of the trip request.

In Example 7, the subject matter of Examples 1-6 includes, a mobile device including the trip planning circuit, the mobile device comprising a smartphone, wherein the trip planning circuit is configured to receive the indication of physiologic ability of the user from the smartphone.

In Example 8, the subject matter of Examples 1-7 includes, a wearable physiological sensor configured to receive one of heart rate information and activity information of the user, wherein the trip planning circuit is configured to receive the indication of physiological ability of the user from the wearable physiological sensor.

In Example 9, the subject matter of Examples 1-8 includes, wherein the trip planning circuit is configured, after the user arrives at the destination, to update a user profile with physiological information of the user associated with the trip request, to anonymize the physiological information of the user associated with the trip request, and to upload the anonymized physiological information to a remote database.

In Example 10, the subject matter of Examples 1-9 includes, wherein the navigation circuit is configured to determine candidate travel paths, wherein each candidate travel path comprises multiple segments, the segments comprising at least one of a walk segment, a bike segment, an e-scooter segment, a vehicle segment (e.g., a ride-sharing vehicle segment, a taxi segment, a partial or fully-autonomous vehicle segment, etc.), a bus segment, or a train segment, wherein the trip planning circuit is configured to determine adjusted scores for each of the candidate travel paths using the indication of physiological ability of the user and the multiple segments of the candidate travel paths, wherein, in response to communicating the at least one of the adjusted scores to the user, the trip planning circuit is configured to receive a selected candidate travel path, and wherein the navigation circuit is configured to route the user to the destination using the selected candidate travel path.

In Example 11, the subject matter of Examples 9-10 includes, wherein the trip planning circuit is configured to receive the indication of physiological ability of the user from the user profile.

Example 12 is a system comprising: means for receiving a trip request to route a user to a destination; means for receiving location information of the user; means for determining candidate travel paths for the trip request using the destination and location information of the user; means for determining a score for each of the determined travel candidate paths; means for receiving an indication of physiological ability of the user, means for determining adjusted scores for each of the candidate travel paths using the received indication of physiological ability of the user; and means for communicating at least one of the adjusted scores to the user.

In Example 13, the subject matter of Example 12 includes, wherein means for determining the score for each of the determined travel candidate paths comprises means for determining an indication of an estimated travel time for each respective candidate travel path for an average user.

In Example 14, the subject matter of Example 13 includes, wherein means for determining adjusted scores for each of the candidate travel paths comprises means for determining an indication of at least one of an adjusted estimated travel time or an estimated difficulty for each respective candidate travel path for the user with respect to the received indication of physiological ability of the user.

In Example 15, the subject matter of Examples 12-14 includes, means for receiving environmental information associated with the trip request comprising at least one of pollution information or weather information associated with a start time and a location of the trip request; and means for determining the adjusted scores for each of the candidate travel paths using the received indication of physiological ability of the user and the environmental information.

In Example 16, the subject matter of Examples 12-15 includes, wherein means for receiving the indication of physiological ability of the user comprises receiving one of heart rate information or activity information of the user using a wearable physiologic sensor.

In Example 17, the subject matter of Example 16 includes, means for updating a user profile with physiological information of the user associated with the trip request after the user arrives at the destination; and means for anonymizing the physiological information of the user associated with the trip request; and means for uploading the anonymized physiological information to a remote database.

Example 18 is at least one non-transitory machine-readable medium including instructions for multimodal trip planning, the instructions, when executed by hardware circuitry, cause the hardware circuitry to perform operations comprising: receiving a trip request to route a user to a destination; receiving location information of the user; determining candidate travel paths for the trip request using the destination and location information of the user; determining a score for each of the determined travel candidate paths; receiving an indication of physiological ability of the user, determining adjusted scores for each of the candidate travel paths using the received indication of physiological ability of the user; and communicating at least one of the adjusted scores to the user.

In Example 19, the subject matter of Example 18 includes, wherein determining the score for each of the determined travel candidate paths comprises determining an indication of an estimated travel time for each respective candidate travel path for an average user.

In Example 20, the subject matter of Example 19 includes, wherein determining adjusted scores for each of the candidate travel paths comprises determining an indication of at least one of an adjusted estimated travel time or an estimated difficulty for each respective candidate travel path for the user with respect to the received indication of physiological ability of the user.

In Example 21, the subject matter of Examples 18-20 includes, the operations comprising: determining the indication of physiological ability of the user using at least one of heart rate or activity information of the user, wherein determining the adjusted scores for each of the candidate travel paths comprises determining the adjusted scores for each of the candidate travel paths as a function of the indication of physiological ability of the user compared to an average physiological ability of a group of users.

In Example 22, the subject matter of Examples 18-21 includes, the operations comprising: receiving environmental information associated with the trip request comprising at least one of pollution information or weather information associated with a start time and a location of the trip request; and determining the adjusted scores for each of the candidate travel paths using the received indication of physiological ability of the user and the environmental information.

In Example 23, the subject matter of Examples 18-22 includes, the operations comprising: receiving the trip request, determining adjusted scores, and communicating at least one of the adjusted scores to the user comprises using a smartphone, and wherein receiving the indication of physiological ability of the user comprises receiving one of heart rate information or activity information of the user using a wearable physiologic sensor.

In Example 24, the subject matter of Examples 18-23 includes, the operations comprising: updating a user profile with physiological information of the user associated with the trip request after the user arrives at the destination; and anonymizing the physiological information of the user associated with the trip request; and uploading the anonymized physiological information to a remote database.

In Example 25, the subject matter of Examples 18-24 includes, wherein each of the candidate travel paths comprise multiple segments, the segments comprising at least one of a walk segment, a bike segment, an e-scooter segment, a vehicle segment (e.g., a ride-sharing vehicle segment, a taxi segment, a partial or fully-autonomous vehicle segment, etc.), a bus segment, or a train segment, wherein determining adjusted scores for each of the candidate travel paths comprises using the indication of physiological ability of the user and the multiple segments of the candidate travel paths, wherein the operations comprise: in response to communicating the at least one of the adjusted scores to the user, receiving a selected candidate travel path, and routing the user to the destination using the selected candidate travel path.

Example 26 is at least one machine-readable medium including instructions that, when executed by processing circuitry, cause the processing circuitry to perform operations to implement of any of Examples 1-25.

Example 27 is an apparatus comprising means to implement of any of Examples 1-25.

Example 28 is a system to implement of any of Examples 1-25.

Example 29 is a method to implement of any of Examples 1-25.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to suggest a numerical order for their objects.

The above description is intended to be illustrative, and not restrictive. For example, the above-described examples (or one or more aspects thereof) may be used in combination with others. Other embodiments may be used, such as by one of ordinary skill in the art upon reviewing the above description. The Abstract is to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. However, the claims may not set forth every feature disclosed herein as embodiments may feature a subset of said features. Further, embodiments may include fewer features than those disclosed in a particular example. Thus, the following claims are hereby incorporated into the Detailed Description, with a claim standing on its own as a separate embodiment. The scope of the embodiments disclosed herein is to be determined with reference to the appended claims, along with the full scope of equivalents to which such claims are entitled.

## Claims

1. A method comprising:
receiving a trip request to route a user to a destination;
receiving location information of the user;
determining candidate travel paths for the trip request using the destination and location information of the user;
determining a score for each of the determined travel candidate paths;
receiving an indication of physiological ability of the user,
determining adjusted scores for each of the candidate travel paths using the received indication of physiological ability of the user; and
communicating at least one of the adjusted scores to the user.

2. The method of claim 1, wherein determining the score for each of the determined travel candidate paths comprises determining an indication of an estimated travel time for each respective candidate travel path for an average user.

3. The method of claim 2, wherein determining adjusted scores for each of the candidate travel paths comprises determining an indication of at least one of the adjusted estimated travel time or an estimated difficulty for each respective candidate travel path for the user with respect to the received indication of physiological ability of the user.

4. The method of any of claims 1 to 3, comprising:
determining the indication of physiological ability of the user using at least one of heart rate or activity information of the user,
wherein determining the adjusted scores for each of the candidate travel paths comprises determining the adjusted scores for each of the candidate travel paths as a function of the indication of physiological ability of the user compared to an average physiological ability of a group of users.

5. The method of any of claims 1 to 4, comprising:
receiving environmental information associated with the trip request comprising at least one of pollution information or weather information associated with a start time and a location of the trip request; and
determining the adjusted scores for each of the candidate travel paths using the received indication of physiological ability of the user and the environmental information.

6. The method of any of claims 1 to 5, comprising:
receiving the trip request, determining adjusted scores, and communicating at least one of the adjusted scores to the user comprises using a smartphone,
wherein receiving the indication of physiological ability of the user comprises receiving one of heart rate information or activity information of the user using a wearable physiologic sensor.

7. The method of any of claims 1 to 6, comprising:
updating a user profile with physiological information of the user associated with the trip request after the user arrives at the destination; and
anonymizing the physiological information of the user associated with the trip request; and
uploading the anonymized physiological information to a remote database.

8. The method of any of claims 1 to 7, wherein each of the candidate travel paths comprise multiple segments, the segments comprising at least one of a walk segment, a bike segment, an e-scooter segment, an autonomous vehicle segment, a taxi segment, a bus segment, or a train segment,
wherein determining adjusted scores for each of the candidate travel paths comprises using the indication of physiological ability of the user and the multiple segments of the candidate travel paths, and
wherein the method further comprises:
in response to communicating the at least one of the adjusted scores to the user, receiving a selected candidate travel path; and
routing the user to the destination using the selected candidate travel path.

9. A system comprising means to perform a method as claimed in any preceding claim.

10. The system of claim 9, comprising:
a trip planning circuit configured to receive a trip request to route a user to a destination, and to receive location information of the user; and
a navigation circuit configured to determine candidate travel paths for the trip request using the destination and location information of the user, and to determine a score for each of the determined travel candidate paths,
wherein the trip planning circuit is configured to receive an indication of physiological ability of the user, and
wherein the trip planning circuit is configured to determine adjusted scores for each of the candidate travel paths using the received indication of physiological ability of the user and to communicate at least one of the adjusted scores to the user.

11. The system of claim 10, comprising:
a physiological ability circuit configured to determine the indication of physiological ability of the user using at least one of heart rate or activity information of the user,
wherein the trip planning circuit is configured to determine adjusted scores for each of the candidate travel paths as a function of the indication of physiological ability of the user compared to an average physiological ability of a group of users.

12. The system of any of claims 10 to 11, wherein the trip planning circuit is configured to receive environmental information associated with the trip request,
wherein the trip planning circuit is configured to determine the adjusted scores for each of the candidate travel paths using the received indication of physiological ability of the user and the environmental information, and
wherein the environmental information comprises at least one of pollution information or weather information associated with a start time and a location of the trip request.

13. The system of any of claims 10 to 12, comprising at least one of a mobile device or a wearable physiological sensor configured to receive one of heart rate information and activity information of the user,
wherein the trip planning circuit is configured to receive the indication of physiologic ability of the user from at least one of the mobile device or the wearable physiological sensor.

14. The system of any of claims 10 to 13, wherein the navigation circuit is configured to determine candidate travel paths, wherein each candidate travel path comprises multiple segments, the segments comprising at least one of a walk segment, a bike segment, an e-scooter segment, an autonomous vehicle segment, a taxi segment, a bus segment, or a train segment,
wherein the trip planning circuit is configured to determine adjusted scores for each of the candidate travel paths using the indication of physiological ability of the user and the multiple segments of the candidate travel paths,
wherein, in response to communicating the at least one of the adjusted scores to the user, the trip planning circuit is configured to receive a selected candidate travel path, and
wherein the navigation circuit is configured to route the user to the destination using the selected candidate travel path.

15. At least one non-transitory machine-readable medium including machine-readable instructions that, when executed by hardware circuitry, cause the hardware circuitry to carry out a method or realise an apparatus as claimed in any preceding claim.
